# EUROPEAN PATENT APPLICATION

(11) **EP 4 186 487 A1**
(43) Date of publication of application: **31.05.2023**
(21) Application number: 22209405.4
(22) Date of filing: 24.11.2022
(51) Int. Cl.: A61K 8/02, A61K 8/19, A61K 8/22, A61K 8/38, A61K 8/73, A61K 8/81, A61Q 11/00

(54) **TOOTH-WHITENERS COMPRISING MODIFIED NIOBIUM COMPOUNDS ASSOCIATED WITH CATIONS, METHOD AND USES**

(30) Priority: 25.11.2021 BR 132021023805
(71) Applicant: Universidade Federal de Minas Gerais, 31270-901 Belo Horizonte (BR); Ondonto Tech Pesquisa e Inovação, Ltda, 30140-128 Belo Horizonte, MG (BR)
(72) Inventor: ALVES DE OLIVEIRA, Luiz Carlos, 31270-901 Belo Horizonte, MG (BR); BELCHIOR, Jadson Cláudio, 31270-901 Belo Horizonte, MG (BR); CHAGAS, Poliane, 31270-901 Belo Horizonte, MG (BR); DE CASTRO OLIVEIRA, Cinthia, 31270-901 Belo Horizonte, MG (BR); DOS SANTOS ALVES, Luís Fernando Morgan, 31270-901 Belo Horizonte, MG (BR); DE SOUSA CANESCHI, Camilia, 31270-901 Belo Horizonte, MG (BR); BENETTI FARIA, Francine, 31270-901 Belo Horizonte, MG (BR)
(74) Representative: Clarke Modet & Co.

(57) **Abstract**

The present technology relates to whitening toothpaste and gels comprising modified niobium compounds associated with cations, method of obtaining and use thereof. The niobium compounds give the product the ability to act in tooth whitening with visible radiation, the radiation can be natural or artificial, without the need to use free peroxides. The mechanisms of whitening action come from electronic excitation, from the incidence of radiation on niobium compounds with cations, which may be due to the action of the functional chemical groups present in the nanoparticles, even in the absence of light. In addition, when dispersed in a toothpaste, the compounds can act as chemical whiteners, in addition to leading to a decrease in caries bacteria due to the oxidant functional groups in the nanoparticles.

## Description

The present patent application is a Certificate of Addition to the patent application BR1020200153676 entitled "TOOTH-WHITENING GEL COMPRISING MODIFIED NIOBIUM COMPOUNDS, METHOD AND USE". The present technology relates to whitening toothpaste and gels comprising modified niobium compounds associated with cations, method of obtaining and use thereof. The niobium compounds give the product the ability to act in tooth whitening with visible radiation, the radiation can be natural or artificial, without the need to use free peroxides. The mechanisms of whitening action come from electronic excitation, from the incidence of radiation on niobium compounds with cations, which may be due to the action of the functional chemical groups present in the nanoparticles, even in the absence of light. In addition, when dispersed in a toothpaste, the compounds can act as chemical whiteners, in addition to leading to a decrease in caries bacteria due to the oxidant functional groups in the nanoparticles.

Tooth whitening is a procedure routinely used in dental offices to improve dental aesthetics. The conventional whitening procedure consists of applying a gel based on hydrogen peroxides or carbamide for better stability. However, whitening from the use of peroxides may be associated with adverse effects, such as structural changes, demineralization of the tooth surface and subsurface, reductions in surface microhardness, and increased surface roughness of enamel and dentin (Chen, Ying- Hui et al. Journal of Dentistry, v. 95, 103318, 2020).

Although the use of tooth whiteners with low concentrations of peroxide represents an advantage as it is less aggressive to exposed tissues, the need to produce tooth whiteners totally free of these components in their formulations is evident.

The scientific literature reports several studies highlighting the importance of minimizing the adverse effects of the use of peroxides or replacing peroxides with other whitening components or by the process of incidence of radiation on photobleaching compounds.

The use of photosensitive materials acting in tooth whitening processes is widely reported in the state of the art, as discussed below.

Patent document US20040191729A1, whose priority date is 11/29/2001, entitled *"Dental phototherapy methods and compositions",* reports the application of a dental bleaching and antimicrobial compound based on a nontoxic chromophore that is activated by radiation in the visible, ultraviolet and infrared regions. The chromophore can act as a dental bleaching and/or antimicrobial agent and can also be applied to treat oral diseases such as caries and periodontitis.

The patent document JP2004292429A, the priority date of which is 03/10/2003, entitled *"Bleaching agent set for teeth and method for bleaching teeth",* describes a tooth whitener with photobleaching action. The whitener described is composed of titanium oxide powder, titanium oxynitride and/or titanium oxide doped with nitrogen dispersed in an organic solvent and must also comprise a thickener. The formation of hydrogen peroxide from the contact of the whitener with water is described, so that the bleaching activity of the product is associated with the presence of hydrogen peroxide combined with the photocatalytic activity of the titanium compounds.

Patent document BR1020200153676, entitled "TOOTH-WHITENING GEL COMPRISING MODIFIED NIOBIUM COMPOUNDS, METHOD AND USE", the priority date of which is 07/28/2020, relates to a whitening gel containing niobium compounds modified by leaching with peroxides dispersed in a polymer matrix. The gel must be applied together with hydrogen peroxide to present the whitening activity. It is worth mentioning that the use of free peroxides in the whitening process induces sensitization of the teeth due to demineralization and damage to tooth enamel and dentin. The association of cation-modified niobium compounds or the photosensitive activity of the material is not mentioned.

The use of niobium nanoparticles modified with cations, the photosensitive niobates, was not found in the state of the art, to promote tooth whitening with the action of visible light from a product totally free of free peroxides.

The present technology relates to tooth whiteners comprising photosensitive niobium compounds modified with cations. Its advantages compared to the state of the art are the absence of free peroxides in the whitening process and the use of radiation in the visible region. The addition of cations to niobium compounds, in adequate concentrations, allows the precipitation of niobium compounds associated with cations, which have functional groups that can act as whiteners even in the absence of radiation and are photosensitive. Thus, from the incidence of radiation, the new cation-associated niobium compounds have their activity to degrade the molecules that darken the teeth increased. In this way, the activity of niobium compounds with cations results in dispensing the use of peroxides during the whitening process. The absence of free peroxides in the product formulation mainly results in the protection of tooth tissues. It is important to emphasize that the radiation used is of high wavelength, reducing operating costs and avoiding damage to the client's health, due to its low energy. Finally, the use of niobates renders these products novel by employing this element outside of metallurgy, where it is mostly used. Brazil has the largest reserves and is the world's largest producer of niobium, thus, its application in several areas is interesting and economically promising.

### BRIEF DESCRIPTION OF DRAWINGS

**Figure 1** shows the whitening process with photosensitive niobates in bovine teeth. In a), the application of niobates on a bovine tooth; in b), the incidence of radiation from the visible region on teeth with niobates; in c), the tooth before whitening; and in d), the tooth after whitening.
**Figure 2** shows a graph in which the whitening effect of a commercial product containing 35% hydrogen peroxide and methylene niobate is quantitatively compared, using ΔE.

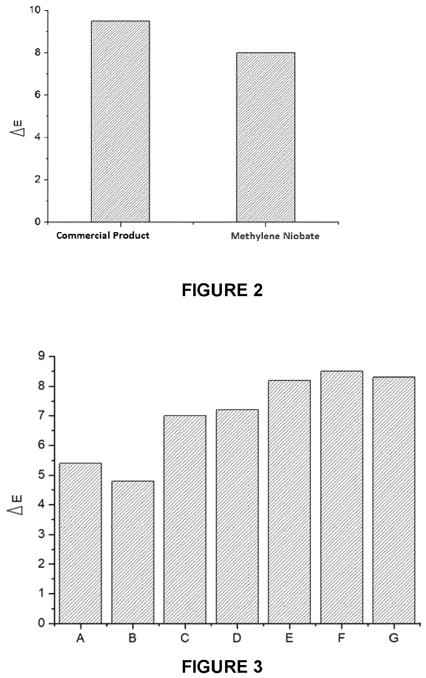

**Figure 3** shows a graph which quantitatively compares by means of ΔE the whitening effect of A, a commercial toothpaste with 2% pf hydrogen peroxide; B, a commercial toothpaste with niobium oxide; C, a toothpaste with solidified niobate without the addition of cation in the ratio of 3% m/m of niobate; D, a toothpaste with solidified niobate with Ca²⁺ in the ratio of 3% m/m of calcium niobate; E, a toothpaste with solidified niobate with Fe³⁺ in the ratio of 3% of iron (III) niobate; F, a toothpaste with niobates obtained from commercial niobium phosphate in the ratio of 1% m/m of niobates; and G, a toothpaste with niobates obtained from commercial niobium phosphate in the ratio of 1% m/m of niobates.

### DETAILED DESCRIPTION OF THE TECHNOLOGY

The present technology relates to toothpaste and gels comprising modified niobium compounds associated with cations, method of obtaining and use thereof. The niobium compounds give the product the ability to act in tooth whitening with visible radiation, the radiation can be natural or artificial, without the need to use free peroxides. The mechanisms of whitening action come from electronic excitation, from the incidence of radiation on niobium compounds with cations, which may be due to the action of the functional chemical groups present in the nanoparticles, even in the absence of light. In addition, when dispersed in a toothpaste, the compounds can act as chemical whiteners, in addition to leading to a decrease in caries bacteria due to the oxidant functional groups in the nanoparticles.

Tooth whiteners comprise nanostructured niobium compounds modified by previous reaction with acids or peroxides and associated with cations, incorporated into toothpastes or polymeric matrices, in the ratio of 0.1 to 30% m/m of niobates in relation to the toothpaste in the ratio of 0.5 to 2% m/m of niobates in relation to the polymer.

The toothpaste used may comprise carboxymethyl cellulose, calcium carbonate, sodium lauryl sulfate and excipients.

The polymer matrix can be selected from the group comprising carbopol and hydroxyethylcellulose.

The niobium compounds used can be selected from the group comprising niobium oxide, niobium pentoxide, niobium ammonium oxalate, niobium chloride and niobium phosphate.

The acids used can be selected from the group comprising oxalic acid, phosphoric acid or hydrofluoric acid, with a concentration of 1 and 70% v/v and the peroxides can be selected from hydrogen peroxide or organic peroxide group, such as benzoyl peroxide or carbamide peroxide in concentrations from 10 to 50% m/v.

The cations associated with modified niobium compounds can be selected from the group comprising Ca²⁺, K⁺, Fe³⁺, Fe²⁺, Mn²⁺, Co²⁺ and cationic methylene blue.

The tooth whitener comprising toothpaste composed of nanostructured niobium is creamy and is indicated for use in oral brushing, resulting in teeth whitening. The tooth whitener comprising niobium compounds and a polymer matrix is in the form of a gel and is indicated for use in tooth whitening with incidence of radiation in the ultraviolet and visible region, at a wavelength of 200 to 800 nm.

Thus, the process for obtaining dental whiteners comprises the following steps:
**a.** Modifying niobium precursors selected from the group comprising niobium oxide, niobium pentoxide, niobium ammonium oxalate and niobium phosphate, alone or in combination, with peroxides or acids in a ratio between 1:10 (peroxide) and 1:20 (acids) of leaching agents in relation to niobium compounds;
**b.** Adjusting the pH of the solution obtained in "a" to values between 1 and 4;
**c.** Stirring the solution obtained in "b", with a speed between 100 and 300 rpm, for a period of 1 to 10 minutes;
**d.** Leaving the solution to rest for a period of 1 to 12 hours;
**e.** Centrifuging the solution obtained in "d", with a speed between 2000 and 3000 rpm, and separating the supernatant comprising modified nanostructured niobium compounds;
**f.** Precipitating the modified nanostructured niobium compounds obtained in "e" from the addition of a cation solution, consisting of cations selected from the group comprising Ca²⁺, Fe³⁺, Fe²⁺, Mn²⁺, Co²⁺ or cationic methylene blue, at a concentration of 100 to 1000 mg/L, to the supernatant obtained in "e";
**g.** Lyophilizing the gel obtained in "f";
**h.** Dispersing the powder obtained in "g" in toothpaste or polymer matrix of carbopol or hydroxyethylcellulose in the ratio between 0.1 and 30% m/m of niobate in relation to the toothpaste or in the ratio between 0.5 and 2% of niobate to the polymer matrix.

The niobium precursors, used to obtain the niobates, described in step "a" can be selected from the group comprising niobium oxide, niobium pentoxide, ammoniacal niobium oxalate, niobium chloride and niobium phosphate. While the acidified solution used for the chemical treatment of step "a" can be selected from oxalic acid, phosphoric acid or hydrofluoric acid, with a concentration between 1 and 70%, or from oxidizing agents with hydrogen peroxide or organic peroxides, such as benzoyl or carbamide peroxide in concentrations from 10 to 50% m/v.

In step "b", the pH adjustment of the solution can be carried out by adding solutions selected from the group comprising oxalic acid, phosphoric acid or hydrofluoric acid, at a concentration of 0.1 to 1 mol.L⁻¹.

From the leaching step of the niobium compounds and the pH control, the agglomeration of the species occurs, forming anionic oligomers comprising niobium. In step "f", niobates are precipitated in the form of nanoparticles by reaction with metallic or organic cations, which can be selected from the group comprising Ca²⁺, Fe³⁺, Fe²⁺, Mn²⁺, Co²⁺ and cationic methylene blue at concentrations between 100 and 1000 mg/l.

The present technology can be better understood through the following non-limiting examples.

### EXAMPLE 1 - OBTAINING PHOTOSENSITIVE NIOBATES

A dispersion of 2.5 g of niobium pentoxide was obtained with the addition of 50.0 mL of distilled water. Soon after, 4.0 mL of a 35% v/v oxalic acid solution were added. The dispersion was kept under constant stirring at 100 rpm for 10 minutes. After this period, the solution was aged for 12 hours and then centrifuged at a speed of 3,500 rpm. The supernatant containing negatively charged niobium oligomers was separated and divided into different aliquots. Solutions containing the following cations separately Ca²⁺, Fe³⁺, Fe²⁺, Mn²⁺ and Co²⁺ were added to each of the supernatant aliquots, at a concentration of 100 mg/L and cationic methylene blue at a concentration of 1,000 mg/L. A gel-like precipitate was formed after the addition of each cation in each aliquot. Afterwards, the gel was lyophilized and, as a result, niobate with powdered cations was obtained. Each cation added to the nanostructured niobate gave rise to a different material and solids were produced with all the mentioned cations.

### EXAMPLE 2 - TEETH WHITENING TESTS WITH BOVINE TEETH USING VISIBLE LIGHT ASSISTED NIOBATES.

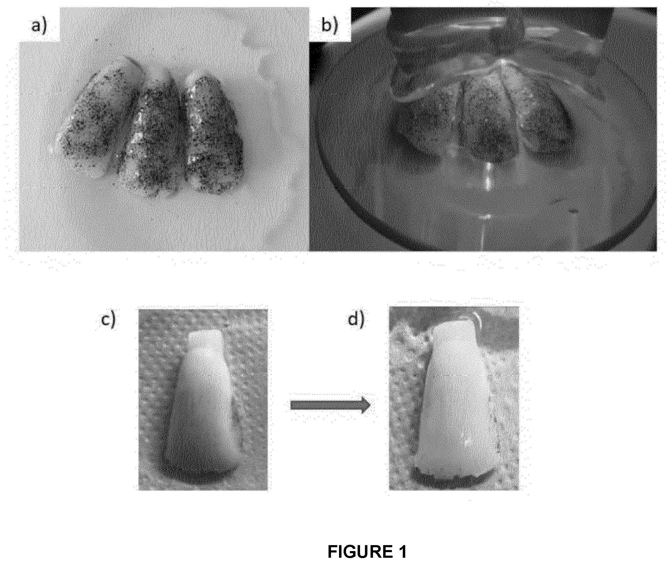

Bovine teeth were cleaned and preserved in thymol solution (0.1%). The initial buccal surface shade for the middle third of each tooth was determined using the VITA Easy Shade spectrophotometer (VITA, Bad Sackingen, Germany). The whitening tests were performed using niobates with methylene, Fe²⁺, Co²⁺ and Mn²⁺ assisted by a source of radiation in the visible region. Figure 1 shows one of the procedures used in the tooth whitening process. For the tooth whitening test, the buccal surface of each specimen was covered with a layer of niobates dispersed in Carbopol. During the experiments, care was taken to keep the teeth hydrated, thus avoiding the already known optical aspect of greater whiteness when dental dehydration occurs. The mediate loss of water from the subsurface of dental enamel alters the light reflection and refraction indices, giving more temporary whiteness that after hydration is lost. Niobate applications were performed in triplicate and the teeth were in contact with the products for 40 minutes. After the exposure time, the teeth were washed and subjected to a new application of the niobium-based material. Four 40-minute applications were made on each tooth. At the end of each application, the tooth color was measured using the VITA Easy Shade spectrophotometer. Figure 1c shows the qualitative result obtained from tooth whitening using methylene niobate under visible light.

Teeth whitening levels were compared using the VITA 3D Master scale (Vita, Bad Sackingen, Germany). Photographs of bovine teeth were recorded before and after tooth whitening using a Canon T6i camera, macro 100mm, manual mode, speed 1/160, diaphragm 5.0, ISO 1600, without the use of flash. The VITA 3D Master dental color scale is a tool to help determine the color of natural teeth. Three parameters are evaluated individually to jointly determine the final color of the teeth. They are, 1) brightness: values from 1 to 5 describe teeth with more brightness and with less brightness. Number 1 represents the one with the greatest brightness; 2) color intensity/luminosity: can also be described as color chroma and is measured on a numerical scale (1, 1.5, 2, 2.5 and 3) that represents colors from pale to intense. Number 1 represents the pale; 3) Color Shade: The L-M-R letters indicate different color intensities. The letter L represents yellowish teeth, the letter R represents reddish teeth and the letter M represents the mixture between yellowish and reddish. To measure the results of this study regarding the changes in each of the three parameters described above, scores were assigned. With each modification of each of the three parameters, one (01) point is assigned or withdrawn in case of evolution or regression of whitening. This analysis allows for the determination of the ΔE, that is, the quantitative difference in whitening obtained. The ΔE measurement is represented in Figure 2.

The ΔE value represents the color variation of bovine teeth, before and after tooth whitening. The effect of niobates on tooth whitening was compared to the commercial product that uses 35% peroxide, since the objective is to replace the commercial product that uses hydrogen peroxide as a bleaching agent. Figure 2 shows that methylene niobate presents efficient teeth whitening results, only slightly lower than the commercial product, which, however, uses a high content of hydrogen peroxide. In addition, methylene niobate presents efficient results in a few tooth whitening sessions and without causing side effects in the patient due to the absence of peroxide. This fact is of high clinical relevance, since the indiscriminate use of high concentrations of peroxides and prolonged application times can cause undesirable damage to the dental structure, ranging from increased sensitivity to pulp necrosis or degradation of the enamel crystalline structure.

Fe, Co or Mn niobates showed similar results. It is important to highlight that tooth whitening using niobates in the presence of radiation proved to be very efficient with few product application sessions, which makes the process faster and less expensive when compared to conventional treatment.

### EXAMPLE 3 - TEETH WHITENING TESTS WITH BOVINE TEETH USING NIOBATES AS TOOTHPASTE COMPONENTS

The niobate formed with Ca²⁺ was incorporated, by dispersion using a stirrer, in a toothpaste free from whitening and abrasive compounds (163 mL Deionized water, 3 g of Carboxymethyl cellulose, 225 g of Calcium carbonate, 5 g of Sodium Lauryl Sulfate, 0.5 g of Nipagim, 0.5 g of Saccharin, Sorbitol) prepared especially for the experiment.

For the whitening test, 6 bovine teeth were used in each experimental group (n = 6). Fourteen 2-minute brushes were performed. Every one minute the tooth was washed and more paste was added to the brush. Each specimen was brushed for 28 minutes.

The color evaluations were performed in triplicate and in two stages: Initial time T(0) and Final time T(f) were measured by Vita Easyshade 5.0, Ultraviolet Visible Reflectance spectrophotometer (VITA Zahnfabrik, Bad Sackingen, Baden-Wurttemberg, Germany), calibrated according to the manufacturer at each reading.

Teeth whitening levels were compared using the VITA 3D Master scale (Vita, Bad Sackingen, Germany). Photographs of bovine teeth were recorded before and after tooth whitening using a Canon T6i camera, macro 100 mm, manual mode, speed 1/160, diaphragm 5.0, ISO 1600, without the use of flash. The VITA 3D Master Dental Color Scale is a tool to help determine the color of natural teeth. Three parameters are evaluated individually to jointly determine the final color of the teeth. They are, 1) brightness: values from 1 to 5 describe teeth with more brightness and with less brightness. Number 1 represents the one with the greatest brightness; 2) color intensity/luminosity: can also be described as color chroma and is measured on a numerical scale (1, 1.5, 2, 2.5 and 3) that represents colors from pale to intense. Number 1 represents the pale; 3) Color Shade: The L-M-R letters indicate different color intensities. The letter L represents yellowish teeth, the letter R represents reddish teeth and the letter M represents the mixture between yellowish and reddish. To measure the results of this study regarding the changes in each of the three parameters described above, scores were assigned. With each modification of each of the three parameters, one (01) point is assigned or withdrawn in case of evolution or regression of whitening. This analysis allows for the determination of the ΔE, that is, the quantitative difference in whitening obtained. The ΔE measurement is represented in Figure 3.

Figure 3 presents comparisons between the quantified ΔE for different samples of toothpaste with niobate and commercial toothpaste containing 2% hydrogen peroxide. Sample A corresponds to the commercial product presenting ΔE = 5.3. Sample B corresponds to the niobium oxide used directly, without the transformation into niobates described in the present application. Sample B showed low bleaching power (ΔE = 4.8), the effect being due to the abrasive effect. Samples C, D, and E correspond to solidified niobate without addition of cation, solidified niobate with Ca²⁺ and solidified niobate with Fe³⁺, all in the ratio of 3% m/m of the material in the toothpaste. Samples F and G correspond to niobates obtained from commercial niobium phosphate in toothpaste, in the ratio of 1% and 2%, respectively. Thus, the compounds obtained to be used as whiteners, samples C, D, E, F and G, whose ΔE values are 6.9; 7.1; 8.1; 8.7 and 8.6, respectively, show whitening results above the commercial compound that claims this property.

The present invention is relevant for the development of materials for dental application based on niobium compounds to produce a toothpaste/paste using commercial or synthetic materials, as an alternative to the materials currently used, mainly in dental offices. Furthermore, because this invention employs a photosensitive compound that, in the presence of light, generates reactive species with a high capacity to oxidize molecules impregnated in the teeth that give it color, favoring tooth whitening. It is important to highlight that the whitening process occurs without the need to use peroxides, eliminating the side effects of the treatment currently used.

## Claims

1. **TOOTH WHITENERS characterized by** comprising nanostructured niobium compounds modified by previous reaction with acids or peroxides and associated with cations, incorporated in toothpastes or polymer matrices, in the ratio of 0.1 to 30% m/m of niobate in relation to the paste, or in the ratio of 0.5 to 2% m/m of niobate in relation to the polymer.

2. **TOOTH WHITENERS, according to claim 1, characterized in that** the niobium compounds are selected from the group comprising niobium oxide, niobium pentoxide, niobium ammonium oxalate and niobium phosphate, the niobium sources being able to be isolated or in combination.

3. **TOOTH WHITENERS, according to claim 1, characterized in that** the acids are selected from the group comprising oxalic acid, phosphoric acid or hydrofluoric acid, with a concentration of 1 to 70% v/v and the peroxides are selected from the group comprising hydrogen peroxide, benzoyl peroxide or carbamide peroxide, with a concentration of 1 to 50% m/v.

4. **TOOTH WHITENERS, according to claim 1, characterized in that** the cations are selected from the group comprising Ca²⁺, K⁺, Fe³⁺, Fe²⁺, Mn²⁺, Co²⁺ and cationic methylene blue.

5. **TOOTH WHITENERS, according to claim 1, characterized in that** the polymer matrix is selected from the group comprising carbopol or hydroxyethylcellulose.

6. **TOOTH WHITENERS, according to claim 1, characterized in that** the toothpaste comprises carboxymethyl cellulose, calcium carbonate, sodium lauryl sulfate and excipients.

7. **METHOD OF OBTAINING TOOTH WHITENERS, defined in claim 1, characterized by** comprising the following steps:
**a)** Modifying niobium precursors selected from the group comprising niobium oxide, niobium pentoxide, niobium ammonium oxalate and niobium phosphate, alone or in combination, with peroxides or acids in a ratio between 1:10 (peroxide) and 1:20 (acids) of leaching agents in relation to niobium compounds;
**b)** Adjusting the pH of the solution obtained in "a" to values between 1 and 4, by adding an acid solution selected from the group comprising oxalic acid, phosphoric acid or hydrofluoric acid in a concentration between 0.1 and 1 mol/L;
**c)** Stirring the solution obtained in "b", with a speed between 100 and 300 rpm, for 10 minutes;
**d)** Leaving the solution to rest for a period of time between 1 and 12 hours;
**e)** Centrifuging the solution obtained in "d", with a speed between 2000 and 3000 rpm, and separating the supernatant comprising modified nanostructured niobium compounds;
**f)** Precipitating the modified nanostructured niobium compounds obtained in "e" from the addition of cation solution to the supernatant obtained in "e";
**g)** Lyophilizing the gel obtained in "f";
**h)** Dispersing the powder obtained in "g" in toothpaste or polymer matrix in the ratio between 0.1 and 30% m/m of niobate in relation to the toothpaste or in the ratio between 0.5 and 2% m/m of niobium compounds to the polymer matrix.

8. **METHOD OF OBTAINING TOOTH WHITENERS, according to claim 7, characterized in that,** in step "a", the acidified solution consists of distilled water and a compound selected from the group comprising oxalic acid, phosphoric acid or hydrofluoric acid, in a concentration of 1 to 70% v/v, and the peroxides are selected from hydrogen peroxide, benzoyl peroxide or carbamide peroxide, with concentrations from 1 to 50% m/v.

9. **USE of the tooth whiteners defined in claim 1, characterized in that** it is for the production of toothpastes for oral brushing and teeth whitening.

10. **USE of the tooth whiteners defined in claim 1, characterized in that** it is for the production of polymer matrix gels for tooth whitening by activation from the incidence of radiation in the visible and ultraviolet region, at a wavelength from 200 to 800 nm.
